# EUROPEAN PATENT SPECIFICATION

(11) **EP 0 232 311 B1**
(45) Date of publication and mention of the grant of the patent: **12.05.1993**
(21) Application number: 86904603.7
(22) Date of filing: 03.07.1986
(51) Int. Cl.: A61K 7/06

(54) **TOPICAL COMPOSITION AND METHOD FOR STIMULATING HAIR GROWTH**
TOPISCHE ZUSAMMENSETZUNG UND VERFAHREN ZUR ANREGUNG DES HAARWUCHSES
COMPOSITION TOPIQUE ET PROCEDE DESTINES A STIMULER LA CROISSANCE DES CHEVEUX

(30) Priority: 18.07.1985 US 757131; 30.04.1986 US 858050
(43) Date of publication of application: 19.08.1987
(73) Proprietor: PROCTOR, Peter H., Bellaire, TX 77401 (US)
(72) Inventor: PROCTOR, Peter H., Bellaire, TX 77401 (US)
(74) Representative: McCall, John Douglas
(86) International application number: US8601393
(87) International publication number: WO8700427

(56) References cited:
- EP-A- 0 027 655
- US-A- 3 551 554
- US-A- 3 896 238
- US-A- 4 139 619
- US-A- 4 367 227
- CHEMICAL ABSTRACTS, vol. 93, no. 8, 25th August 1980, page 505, abstract no. 79872n, Columbus, Ohio, US; & JP-A-8022644

## Description

This invention relates to a composition and method for the cosmetic treatment of baldness, particularly androgenic alopecia.

Various treatments were available for conditions such as male and female pattern baldness and alopecia areata. Several substances were known to be effective when administered internally, but had undesirable concomitant systemic effects and the hypertrichosis was not confined to the scalp area. In an effort to avoid these side effects and to confine the hypertrichosis to the scalp area, several attempts were made to apply such substances in a topical preparation to the affected area. However, such attempts had generally been only marginally successful, and the results obtained with the topical preparation containing the orally effective substances were comparable to and generally little better than those obtained with topical application of the carrier only.

U.S. Patent 4,139,619 described a process for stimulating the growth of mammalian hair by the application of 6-amino-4-(substituted amino)-1,2-dihydro-1-hydroxy-2-iminopyrimidines to mammalian skin in association with a topical pharmaceutical carrier.

U.S. Patent 4,347,245 described a composition containing spironolactone in a liquid carrier such as alcohol, urea, mineral oil or white petrolatum.

Stewart, M.E. et al "Antiandrogens and the Skin," International Journal of Dermatology, Vol. 17, pp. 167-179 (1978) described the application to the foreheads of acne patients of 10% cyproterone in 50% aqueous dimethyl sulfoxide, with no reduction in sebum secretion or improvement in acne being produced.

U.S. Patent 4,367,227 described a composition for reducing sebum secretion when applied to the skin, which composition contained cyproterone acetate dissolved in a C₂-C₃ aliphatic alcohol.

According to the present invention, there is provided a composition for topical application to the skin to stimulate hair growth, characterized in that it contains:
(a) from 0.1 to 20 percent by weight of the composition of a pharmacologically acceptable hair growth stimulant compound forming a stable free radical and selected from 5,5-diaryl-hydantoins;
(b) from 5 to 25 percent by weight of the composition of a synergistically effective proportion of a pharmaceutically acceptable hydroxyl radical scavenger; and
(c) a pharmaceutical carrier in which said hair growth stimulant and said scavenger are substantially homogenously dispersed.

In another aspect, the invention is a method of stimulating the growth of hair for cosmetic treatment by applying to the skin to be treated a composition of the present invention. Optimally, the composition of the present invention applied includes an antiandrogen which interferes with the binding of dihydrotestosterone to receptors.

The carrier of the composition, in which the 5,5-diarylhydantoin, DMSO and any antiandrogen will be substantially homogenously dispersed, is preferably an occlusive or semi-occlusive preparation which may be a water-in-oil emulsion, but is most preferably an oil-in-water emulsion. As used herein, the terms "occlusive" or "semi-occlusive" are used in reference to a carrier which substantially prevents or inhibits, respectively, evaporation of water from the skin to which it is applied. Suitable water-in-oil emulsions are commercially available under the trade marks:- Aquaphor, cold cream, Eucerin, hydrous lanolin, Hydrosorb, hydrophilic petrolatum, Nivea, Polysorb, Qualatum and Velvachol. Suitable oil-in-water emulsions are available commercially under the trade marks:-acid mantle cream, Almay emulsion cream, Cetaphil, Dermabase, Dermovan, hydrophilic ointment, Keri cream, Lubriderm cream, Multibase cream, Neobase cream, Univase cream, Vanibase cream, and Wibi. The carrier may further contain various other emollients, emulsifiers, water, perfumes, colorants and preservatives. In a preferred embodiment, the carrier comprises the Dermovan® emulsion, propylene glycol and water.

The 5,5-diaryl-hydantoin is preferably present in the composition in an amount of from 0.5 to 3 percent by weight of the composition and more preferably about 2 percent by weight.

A second essential ingredient is DMSO or a substantially equivalent hydroxyl radical scavenger in a synergistically effective amount. Hydroxyl radical scavengers are, for example, sulfoxides, purines, pyrimidines, thiols, alcohols, halide ions and aromatic hydrocarbons. Hydroxyl radical scavengers suitable in the composition of the present invention are those pharmaceutically acceptable hydroxyl radical scavengers which have a hydroxyl radical scavenger effectiveness substantially equivalent to or better than DMSO. Preferred hydroxyl radical scavengers are the alkyl methyl sulfoxides in which the alkyl substituent has from one to 14 carbon atoms and the β-hydroxyalkyl methyl sulfoxides in which the hydroxyalkyl substituent has from two to 14 carbon atoms, with dimethyl sulfoxide being particularly preferred. Specific representative examples of such sulfoxides include, in addition to DMSO, hexyl methyl sulfoxide, decyl methyl sulfoxide, dodecyl methyl sulfoxide, tetradecyl methyl sulfoxide β-hydroxydecyl methyl sulfoxide, β-hydroxydodecyl methyl sulfoxide and β-hydroxytetradecyl methyl sulfoxide. For convenience, reference is made hereinbelow to DMSO, but it is to be understood that other suitable hydroxyl radical scavengers may be present,partially or entirely, in lieu of DMSO.

The hydroxyl radical scavenger is present in the composition in a proportion effective to, synergistically with the 5,5-diaryl hydantoin, stimulate the growth of hair. For the sulfoxides such as DMSO this amount is generally from 5 to 25 percent by weight of the composition, preferably 15-20 percent by weight of the composition. Depending on the particular carrier, the amount of DMSO present may be adjusted to avoid phase separation.

It has also been found that the hair growth stimulation effected by the present composition is improved when an antiandrogen which interferes with the binding of dihydrotestosterone to receptors in hair follicles is present. These compounds function primarily to block dihdrotestosterone receptors rather than to inhibit the reduction of testosterone. Exemplary of such antiandrogens are spironolactone, cyproterone and cyproterone acetate.

Effective amounts of the antiandrogen generally range from about 0.01 to 5 percent by weight of the composition, but more or less than this may be used depending on the particular antiandrogen. The optimum amount is about 0.01 percent by weight of the composition for spironolactone, about 0.1 percent for cyproterone, and about 0.1 percent for cyproterone acetate. Quite surprisingly, at amounts above these optimums, the effect of the antiandrogens is not as great, and for unknown reasons, in some cases the presence of the antiandrogen in the composition in amounts in substantial excess of the optimum results in a reduced effectiveness in stimulating hair growth in comparison to the composition containing no antiandrogen.

According to the method of the invention, the composition of the invention described above is applied topically to the skin to be treated, such as the scalp. Preferably, the application is once a day with a sufficient amount of the composition to cover the area at which the stimulation of hair growth is desired. It has been found that results are improved when the composition is applied after water-soaking the skin. Thus, a preferred embodiment of the method is convenient in that the composition can be applied once daily immediately following bathing.

Generally, best results are obtained in treatment of bald or thinly-haired scalp areas in which hair loss has not occurred for a period of time substantially in excess of about 3-5 years. The effectiveness also depends, although to a lesser degree, inversely on the age of the user.

Quite surprisingly, it has been discovered that the presence of DMSO in the topically applied pharmaceutical preparation of this invention results in a marked enhancement in the treatment of balding.
While DMSO has been present in topically applied prior art preparations to improve percutaneous penetration of pharmacologically active substances the concentration of DMSO in water must be above about 50 percent by weight before any improvement in penetration is obtained. Because DMSO is effective to produce hypertrichosis when present in the 5,5-diaryl-hydantoin containing composition of this invention at concentrations much less than 50 percent by weight, although not presently fully understood, it is believed that its synergistic effect is due to a pharmacological effect of DMSO, such as, for example, its property as a potent hydroxyl radical scavenger,rather than any effect on increased absorption of 5,5-diaryl-hydantoins.

It is also quite surprising that the presence of DMSO in the present composition generally results neither in irritation of the skin to which the composition is applied (in about 99 percent of patients) nor in an unpleasant odor or taste being experienced, in sharp contrast to heretofore known DMSO-containing preparations.

Application of the composition of this invention to the bald or thinly-haired scalp has been observed to be effective to substantially restore the normal growth of hair in about 3-6 months in about 90 percent of patients treated who had a history of hair loss of less than 5 years. The treatment is substantially free of systemic side effects and the hypertrichosis is confined to the area of application.

The emulsion in which the hair growth stimulant, hydroxyl radical scavenger and any antiandrogen will generally be substantially homogenously dispersed, is preferably an occlusive or semi-occlusive preparation which may be a water-in-oil emulsion, but is most preferably an oil-in-water emulsion. Suitable water-in-oil emulsions are commercially available under the trade names:- Aquaphor, cold cream, Eucerin, hydrous lanolin, Hydrosorb, hydrophilic petrolatum, Nivea, Polysorb, Qualatum and Velvachol. Suitable oil-in-water emulsions are available commercially under the trade names:-acid mantle cream, Almay emulsion cream, Cetaphil, Dermabase, Dermovan, hydrophilic ointment, Keri cream, Lubriderm cream, Multibase cream, Neobase cream, Univase cream, Vanibase cream, and Wibi. The emulsion may further contain various other emollients, emulsifiers, perfumes, colorants and preservatives. In a preferred embodiment, the emulsion comprises the Dermovan emulsion, propylene glycol and water. 5,5-diphenyl-hydantoins and related compounds for use in the present invention are described in U.S.patent 2 409 754.

It is believed that the other pharmacologically acceptable substances which form stable free radicals may also be hair growth stimulants suitably substituted for minoxidil. Free radicals are generally short-lived reactive chemical species having one or more electrons with unpaired spins. However, stable radicals can be formed in some species due to steric protection, resonance stabilization and other means of protecting unpaired electrons.

## Claims

1. A composition for topical application to the skin to stimulate hair growth, containing:
(a) from 0.1 to 20 percent by weight of the composition of a pharmacologically acceptable hair growth stimulant compound forming a stable free radical and selected from 5,5-diaryl-hydantoins;
(b) from 5 to 25 percent by weight of the composition of a synergistically effective proportion of a pharmaceutically acceptable hydroxyl radical scavenger; and
(c) a pharmaceutical carrier in which said hair growth stimulant and said scavenger are substantially homogenously dispersed.

2. A composition according to claim 1, in which said hydantoin is 5,5-diphenyl hydantoin.

3. A composition according to claim 1 or 2, in which the composition comprises from 0.01 to 5 percent by weight of the composition of an antiandrogen which interferes with the binding of dihydrotestosterone to receptors.

4. A composition according to claim 3, in which said antiandrogen is selected from the group consisting of: spironolactone, cyproterone, cyproterone acetate, and combinations thereof.

5. A composition according to any one of the preceding claims in which said stable free radical forming compound is present in an amount of from 0.5 to 3 percent by weight of the composition.

6. A composition according to any one of the preceding claims, in which said stable free radical forming compound is present in an amount of 2 percent by weight of the composition.

7. A composition according to any one of the preceding claims, in which said hydroxyl radical scavenger is selected from the group consisting of: alkyl methyl sulfoxides in which the alkyl substituent has from one to 14 carbon atoms, β-hydroxyalkyl methyl sulfoxides in which the hydroxyalkyl substituent has from two to 14 carbon atoms and combinations thereof.

8. A composition according to any one of the preceding claims, in which said hydroxyl radical scavenger is present in an amount of from 15 to 20 percent by weight of the composition.

9. A composition according to any one of the preceding claims, in which said hydroxyl radical scavenger is dimethyl sulfoxide.

10. A composition according to any one of the preceding claims, in which said carrier is an occlusive or semiocclusive carrier selected from the group consisting of water-in-oil emulsions and oil-in-water emulsions.

11. A composition according to any one of the preceding claims for use in the treatment of baldness.

12. A composition according to any one of the preceding claims for use in the treatment of androgenic alopecia.

13. A method of stimulating hair growth for cosmetic treatment, comprising topically applying to the skin a composition according to any one of the claims 1 to 12.

14. A method according to claim 13, which further comprises water soaking said skin just prior to said application of said composition.

15. A method according to claim 13 or 14, in which 0.5 ml of said composition is applied to the skin once a day.

16. A method according to any one of claims 13 to 15, in which said composition is applied to the scalp.

## Patentansprüche

1. Zusammensetzung für topische oder äußere Auftragung auf die Haut, um Haarwachstum anzuregen, mit:
(a) einer pharmakologisch akzeptierbaren, Haarwachstum anregenden Verbindung, die ein stabiles, freies Radikal bildet und aus 5,5-Diaryl-Hydantoinen ausgewählt ist, mit 0,1 bis 20 Gewichtsprozent der Zusammensetzung;
(b) einem synergetisch wirksamen Anteil eines pharmazeutisch akzeptierbaren Hydroxylradikalfängers mit 5 bis 25 Gewichtsprozent der Zusammensetzung; und
(c) einem pharmazeutischen Trägerstoff, in dem das Haarwachstum anregende Mittel und der Radikalfänger im wesentlichen homogen dispergiert sind.

2. Zusammensetzung nach Anspruch 1, in der das Hydantoin 5,5-Diphenylhydantoin ist.

3. Zusammensetzung nach einem der Ansprüche 1 oder 2, bei der die Zusammensetzung ein die Bindung von Dihydrotestosteron an Rezeptoren behinderndes Antiandrogen mit 0,01 bis 5 Gewichtsprozent der Zusammensetzung aufweist.

4. Zusammensetzung nach Anspruch 3, in der das Antiandrogen aus der Gruppe mit Spyronolaceton, Cyproteron, Cyproteronacetat und Kombinationen derselben ausgewählt ist.

5. Zusammensetzung nach einem der vorstehenden Ansprüche, bei der die stabile, freie Radikale bildende Verbindung in einer Menge von 0,5 bis 3 Gewichtsprozent der Zusammensetzung vorhanden ist.

6. Zusammensetzung nach einem der vorstehenden Ansprüche, bei der die stabile, freie Radikale bildende Verbindung in einer Menge von 2 Gewichtsprozent der Zusammensetzung vorhanden ist.

7. Zusammensetzung nach einem der vorstehenden Ansprüche, bei der der Hydroxylradikalfänger aus der Gruppe mit Alkylmethylsulfoxiden, in denen der Alkylsubstituent 1 bis 14 Kohlenstoffatome aufweist, β-Hydroxylalkylmethylsulfoxiden, bei denen der Hydroxylalkylsubstituent 2 bis 14 Kohlenstoffatome aufweist, und Kombinationen derselben ausgewählt ist.

8. Zusammensetzung nach einem der vorstehenden Ansprüche, bei der der Hydroxylradikalfänger in einer Menge von 15 bis 20 Gewichtsprozent der Zusammensetzung vorhanden ist.

9. Zusammensetzung nach einem der vorstehenden Ansprüche, bei der der Hydroxylradikalfänger Dimethylsulfoxid ist.

10. Zusammensetzung nach einem der vorstehenden Ansprüche, bei der der Träger ein okkludierender oder halbokkludierender Träger ist, der aus der Gruppe mit Wasser-in-Öl-Emulsionen und Öl-in-Wasser-Emulsionen ausgewählt ist.

11. Zusammensetzung nach einem der vorstehenden Ansprüche, zur Verwendung bei der Behandlung von Kahlheit.

12. Zusammensetzung nach einem der vorstehenden Ansprüche, zur Verwendung bei der Behandlung von durch männliche Hormone bedingte Haarlosigkeit.

13. Verfahren zum Stimulieren von Haarwachstum zur kosmetischen Behandlung, das örtliches Auftragen einer Zusammensetzung gemäß einem der Ansprüche 1 bis 12 auf die Haut umfaßt.

14. Verfahren nach Anspruch 13, ferner umfassend ein Waschen der Haut mit Wasser und Seife direkt vor der Anwendung der Zusammensetzung.

15. Verfahren nach Anspruch 13 oder Anspruch 14, bei dem 0,5 ml der Zusammensetzung einmal pro Tag auf die Haut aufgetragen werden.

16. Verfahren nach einem der Ansprüche 13 bis 15, bei dem die Zusammensetzung auf die Kopfhaut aufgetragen wird.

## Revendications

1. Composition pour application locale à la peau pour stimuler la croissance pileuse, contenant :
(a) 0,1 à 20 pour cent en poids, par rapport au poids de la composition, d'un composé pharmaceutiquement acceptable stimulant la croissance pileuse, formant un radical libre stable et choisi parmi les 5,5-diaryl-hydantoïnes ;
(b) 5 à 25 pour cent en poids, par rapport au poids de la composition, d'une proportion à effet synergique d'un fixateur de radicaux hydroxyle pharmaceutiquement acceptable ; et
(c) un support pharmaceutique dans lequel ledit stimulant de croissance pileuse et ledit fixateur sont dispersés de façon sensiblement homogène.

2. Composition selon la revendication 1, dans laquelle ladite hydantoïne est la 5,5-diphényl-hydantoïne.

3. Composition selon la revendication 1 ou 2, dans laquelle la composition comprend 0,01 à 5 pour cent en poids, par rapport au poids de la composition, d'un agent antiandrogène qui s oppose à la liaison de la dihydrotestostérone aux récepteurs.

4. Composition selon la revendication 3, dans laquelle ledit agent antiandrogène est choisi dans le groupe formé par : la spironolactone, la cyprotérone, l'acétate de cyprotérone et leurs associations.

5. Composition selon l'une quelconque des revendications précédentes, dans laquelle ledit composé formant un radical libre stable est présent en une quantité de 0,5 à 3 pour cent en poids de la composition.

6. Composition selon l'une quelconque des revendications précédentes, dans laquelle ledit composé formant un radical libre stable est présent en une quantité de 2 pour cent en poids de la composition.

7. Composition selon l'une quelconque des revendications précédentes, dans laquelle ledit fixateur de radicaux hydroxyle est choisi dans le groupe formé par : les alkylméthylsulfoxydes dans lesquels le substituant alkyle compte 1 à 14 atomes de carbone, les β-hydroxyalkylméthylsulfoxydes dans lesquels le substituant hydroxyalkyle compte 2 à 14 atomes de carbone, et leurs associations.

8. Composition selon l'une quelconque des revendications précédentes, dans laquelle ledit fixateur de radicaux hydroxyle est présent en une quantité de 15 à 20 pour cent en poids de la composition.

9. Composition selon l'une quelconque des revendications précédentes, dans laquelle ledit fixateur de radicaux hydroxyle est le diméthylsulfoxyde.

10. Composition selon l'une quelconque des revendications précédentes, dans laquelle ledit support est un support occlusif ou semi-occlusif choisi dans le groupe formé par les émulsions du type eau dans l'huile et les émulsions du type huile dans l'eau.

11. Composition selon l'une quelconque des revendications précédentes, pour son utilisation dans le traitement de la calvitie.

12. Composition selon l'une quelconque des revendications précédentes, pour son utilisation dans le traitement de l'alopécie androgénique.

13. Procédé de stimulation de la croissance pileuse pour un traitement cosmétique, comprenant l'application locale à la peau d une composition selon l'une quelconque des revendications 1 à 12.

14. Procédé selon la revendication 13, qui comprend de plus un trempage de la peau avec de l'eau immédiatement avant ladite application de ladite composition.

15. Procédé selon la revendication 13 ou 14, dans lequel une quantité de 0,5 ml de ladite composition est appliquée à la peau une fois par jour.

16. Procédé selon l'une quelconque des revendications 13 à 15, dans lequel ladite composition est appliquée au cuir chevelu.
